(19) Europäisches Patentamt / European Patent Office / Office européen des brevets

(11) **EP 3 615 682 B1**

(12) **EUROPEAN PATENT SPECIFICATION**

(45) Date of publication and mention
of the grant of the patent:
**01.04.2026 Bulletin 2026/14**

(21) Application number: **17780202.2**

(22) Date of filing: **13.07.2017**

(51) International Patent Classification (IPC):
**G16B 20/20** (2019.01)   **C12Q 1/6886** (2018.01)

(52) Cooperative Patent Classification (CPC):
**C12Q 1/6886; G16B 20/20;** C12Q 2600/156

(86) International application number:
**PCT/IB2017/054231**

(87) International publication number:
**WO 2018/197934 (01.11.2018 Gazette 2018/44)**

(54) **METHOD FOR SEARCHING AND IDENTIFYING A GENETIC CONDITION PRODROMAL OF THE ONSET OF SOLID TUMORS**

VERFAHREN ZUM SUCHEN UND IDENTIFIZIEREN EINES GENETISCHEN ZUSTANDS ALS VORZEICHEN DES BEGINNS VON SOLIDEN TUMOREN

PROCÉDÉ DE RECHERCHE ET D'IDENTIFICATION D'UNE CONDITION GÉNÉTIQUE PRODROMALE DE L'APPARITION DE TUMEURS SOLIDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.04.2017 IT 201700045353**

(43) Date of publication of application:
**04.03.2020 Bulletin 2020/10**

(73) Proprietor: **Bioscience Services S.r.l.**
**47891 Falciano (SM)**

(72) Inventor: **MUCCI, Giuseppe**
**47891 Falciano (SM)**

(74) Representative: **Ruzzu, Giammario**
**Via Gulli, 5**
**40068 San Lazzaro di Savena (BO) (IT)**

(56) References cited:
**WO-A1-2017/181146       US-A1- 2013 143 747**
**US-A1- 2016 102 358       US-A1- 2016 130 648**

- **JEFFREY GAGAN ET AL: "Next-generation sequencing to guide cancer therapy", GENOME MEDICINE, vol. 7, no. 1, 29 July 2015 (2015-07-29), XP055421971, DOI: 10.1186/ s13073-015-0203-x**
- **DE MATTOS-ARRUDA LETICIA ET AL: "Cell-free circulating tumour DNA as a liquid biopsy in breast cancer", MOLECULAR ONCOLOGY, ELSEVIER, AMSTERDAM, NL, vol. 10, no. 3, 17 December 2015 (2015-12-17), pages 464 - 474, XP029432258, ISSN: 1574-7891, DOI: 10.1016/ J.MOLONC.2015.12.001**
- **MARK SAUSEN ET AL: "Clinical implications of genomic alterations in the tumour and circulation of pancreatic cancer patients", NATURE COMMUNICATIONS, vol. 6, no. 7686, 7 July 2015 (2015-07-07), pages 1 - 6, XP055326405, DOI: 10.1038/ncomms8686**

**Description**

*TECHNICAL FIELD*

The present invention generally refers to the field of cancer prevention and more in detail to solid tumors prevention.

In particular, the invention includes a method for searching and identifying, in an individual, a genetic condition that is prodromal of the onset of solid tumors, and the statistical prediction of the presence of significant possibility of contracting such tumors in individuals considered at risk.

*BACKGROUND ART*

A solid tumor is made up of one or more masses of tissue consisting of tumor cells and stroma (in turn composed of different types of cells and extracellular matrix), characterized by an abnormal and uncontrolled growth, which in some cases can cause a systemic pathology.

The tumors are pathologies caused by genetic alterations; they develop in different steps, consecutive in time, in which a series of subsequent mutations accumulate in the genetic heritage of one or more cells.

A genetic instability condition, associated to an increase of the genetic alterations of the subject is a marker of particular risk for tumor formation. These genetic alterations increase the risk for the carrier cells to cause tumor cells lines.

This process has been thoroughly studied for the colorectal cancers. In the genesis of these tumors, the first mutation, that involves genes called gatekeepers/caretakers, responsible for the control of the genetic stability, causes a selective advantage in the growth of a normal epithelial cell, allowing it to dominate the surrounding cells and become a microscopic clone.

The best known of the gatekeeper genes in the colon is the APC gene.

Almost all (about 80%) the colorectal tumors are characterized by a mutation of the APC gene. The small adenoma caused by this mutation grows very slowly, however, if a second mutation occurs in another gene, as for example the KRAS gene (or ATK1, etc.), this triggers a new phase of clonal growth which causes the expansion of the number of cells involved. The cells with only the APC mutation can remain in the adenoma, but their number is limited with respect to those with mutations in both (or more) genes.

This process of successive mutations, followed by corresponding clonal expansions, continues with mutations in other genes, such as PIK3CA, SMAD4 and TP53, with high probability to generate a malignant neoplasm, which can extend through the underlying basal membrane and metastasize, first generally toward the regional lymph nodes and then to distant organs, such as liver or lungs.

During the last decade, a thorough series of sequencing disclosed the genomic panorama of the most common forms of human cancer. Sequencing of the genome of many tumors has given information related to thousands of mutations and other genomic alterations. At present, more than ten thousand tumor genomes have been sequenced and many other tumor genomes will be characterized in the near future, with gradual reduction of the sequencing costs.

Sequencing of the tumor genomes has allowed different "genes with driver mutations" to be classified and will allow classification of many others. A driver mutation is a mutation within a gene which provides a significant advantage in the growth.

Up to now, the carried out studies have allowed identification of about 140 genes, which, when mutated, can facilitate or "drive" the oncogenesis. A typical tumor can generally contain two to eight such mutations in "driver genes". The remaining mutations are to be considered transitory and they do not offer any advantage of cell growth.

The driver genes are often involved in the regulation of the key molecular pathways of the cell, which regulate, in different forms and modes, three main cellular processes: the cellular differentiation, survival (through pro- or anti-apoptotic signals) and maintenance. At present, one of the most pressing needs in the basic cancer research is a deep understanding of such different pathways. However, even the degree of understanding reached so far in the structure of the tumor genomes is sufficient to guide some current therapeutic choices and has determined the development of more effective approaches in the reduction of cancer morbidity and mortality.

The screening and early diagnosis programmes play an important role in improving healthy survival and reducing mortality in cancer patients. Since non invasive approaches for early diagnosis contribute to encourage the patient's cooperation, it is definitely advisable to include them in screening and prevention programmes.

The increasing knowledge of the molecular pathogenesis of the tumor pathologies and the rapid development of new techniques of molecular analysis are contributing to the development and achievement of identification and analysis of the early molecular alterations in the body fluids. Extracellular DNA ("cell-free DNA" or "cfDNA") can be found in the serum, plasma, urine and other body fluids. Therefore, sampling and molecular analysis of the cfDNA represents a kind of "liquid biopsy" which can constitute a kind of "circulating image" of various specific pathologies.

In the blood, the apoptosis seems to be the most frequent process that generates cfDNA, although in the cancer patients, the portion provided by necrotic processes cannot be completely neglected. An interesting study analyzing cfDNA from the plasma of 32 patients affected with stage 4 colorectal cancer has shown that in 34.4% of the patients the taken DNA had two magnitude dimensions (166bp and 332bp).

**Stroun** *et al.* have described that different cancer alterations can be identified in the cfDNA of a patient. Various articles published afterwards have confirmed that the cfDNA contains specific alterations correlated to the presence of tumors, such as mutations, methylations and variations of the number of copies ("copy number variations" or CNVs) of specific genes, directly referable to tumor cells. thus confirming the existence of the circulating tumor DNA (ctDNA).

Various studies, aimed at correlating the selected samples of tissue and plasma, have been carried out in order to confirm that the analysis of the circulating cfDNA can be used as diagnostic instrument.

An evaluation with NGS techniques (*next-generation sequencing*) performed on 50 tumor genes, that covers 2,800 COSMIC mutations (*Catalogue Of Somatic Mutations In Cancer - http://cancer.sanger.ac.uk/cosmic*) in 60 tumor tissues and 31 plasma samples from 17 patients with metastatic breast tumor has revealed a 76% concordance between tissue and plasma. From these data the authors have drawn a conclusion that plasma can be considered the biological sample adopted for the screening of tumors as a substitute of the metastatic biopsy.

The above mentioned results have been confirmed in a group of 34 patients suffering from 18 different types of tumor: the analysis has involved 46 genes and covered 6,800 COSMIC mutations in samples of tissue and plasma. Twenty seven out of thirty four patients have shown a 97% agreement between the mutations found in the tissue samples and those found in the ctDNA. Consequently, the ctDNA-based NGS analysis could revolutionize the management of patients suffering from cancer pathologies which are potentially curable or metastatic.

Document US 2016/0102358 discloses a method of assessing an individual subject's risk of developing different types of cancer by identifying significant statistical association between multiple genetic markers and cancer risk for a variety of different cancers. In particular, US 2016/0102358 provides a method of producing a personalized cancer risk report for a subject by determining from a nucleic acid sample of the subject a genotype at a plurality of biallelic polymorphic loci. The risk is calculated based on the genotype determined for each plurality of biallelic polymorphic loci.

## *TECHNICAL PROBLEM*

The evaluation of the risk status constitutes an essential component of the testing procedures and genetic analysis. There is a strong likelihood that in the very near future this type of approach will be implemented in a systematic manner in the prior assessment of the cancer pathologies.

On the other hand, it is more and more frequent that "healthy" persons ask to have genetic tests for predisposition to serious pathologies or detecting a pre-pathological status. Also for this reason the physicians should introduce techniques for the definition and management of risk, and for identifying surely or very probably pre-pathological conditions, in their routine monitoring programmes.

The genetic risk refers to the likelihood of an individual carrier of a mutation associated specifically to a determined pathology - in particular as regards the present disclosure, a cancer pathology - actually developing this pathology. On the other hand, the identification of a pre-pathological condition or a condition prodromal of the onset of a pathology relates to the detection of biomolecular signals indicative of a genetic instability situation, which, due to a subsequent evolution, can cause over time, certainly or very likely, the onset of the pathology.

Given that, as already pointed out several times in the text, the carcinogenesis is influenced by both environmental factors and hereditary predisposition, the genetic background associated with a specific disorder considerably affects the definition of the risk correlated to this particular disorder.

According to Wang E. et al. an algorithm based on a series (network) of characteristic elements can be adopted to generate a genetic model of the key components of the tumor and to connect mutating genotypes with clinical phenotypes. The use of this pattern (and others similar) illustrates the strategies for the prediction of possible tumor therapeutic targets, probability of recurrence and risk of the onset of the tumor on the basis of an individual profile of the patient's genomic sequence.

To sum up, prediction methods deriving from a model based on a network of characteristic elements can be used in the diagnosis and optimized management and prevention of the cancer pathologies programmes.

## *OBJECTS OF THE INVENTION*

The main object of the invention is to provide a monitoring method capable of obtaining an evaluation, for a clinically healthy individual, of his/her entry into a genetic condition prodromal of the onset of one form of solid tumor, that is a condition that, sooner or later, almost certainly will make the individual develop the form of tumor being monitored.

## SUMMARY OF THE INVENTION

The invention is set out in the appended set of claims.

This and other objects are obtained by the invention, which relates to a method (based on an algorithm and an associated database) for searching and identifying, in a healthy individual, a genetic condition that is prodromal of development of solid tumors, with the exception of the brain tumors. This method defines the above mentioned condition on the basis of a series of periodical evaluation cycles about the mutation frequency that involve a panel of genes chosen from those associated to the onset of the cited solid tumors.

The method includes isolating DNA from a biological sample which has been taken from an individual (constituted by body fluids, such as blood, urine or spinal fluid), amplification of the DNA and sequencing thereof. A subsequent analysis step includes evaluation of the presence of one or more mutations that involve the cited panel of genes, chosen from a list of known mutations for the monitored genes and indicative of an evolution toward the formation of neoplastic cells.

In particular, an evaluation with NGS techniques (next-generation sequencing) is performed on 50 genes, and a total of 2,800 mutations classified in the COSMIC database (*Catalogue Of Somatic Mutations In Cancer - http://cancer.sanger.ac.uk/cosmic*).

Every evaluation cycle detects, for every monitored gene, the existence of mutations involving it, with particular attention to mutations which occur in known hotspots (positions frequently mutated in cancer patients). In the case of a mutation, it is checked if this detected mutation has already been found in previous evaluation cycles and at which level (mutated percentage or fraction - allelic frequency). In negative case, the new mutation involving this gene is registered and an algorithm calculates a value indicative of the trend that the frequency of mutation shows over time. This value constitutes the "Key Risk Indicator" of the system. The trend is represented with a numerical value, obtained by calculating a relation between the last value of the mutation frequency and the values obtained in the previous evaluation cycles, and its tendency can be represented in a diagram to provide indications of the level of the genetic stability or instability.

Once it has been detected that the threshold level defined for the frequency of mutation is exceeded, according to the identification method of the invention, a report is issued about the entry in a genetic instability path, during which further mutations will make the individual develop a solid tumor over time, certainly or very likely.

The panels of genes and their mutations taken into consideration for every evaluation cycle can include again the whole range of 50 genes associated with the onset of solid tumors, or only some genes and hotspots associated only with one or more chosen tumors.

In particular, it is possible to monitor the individual's genetic situation, whose instability is associated with the onset of only one type of tumor or a single family of tumors. In this case, the number of genes being analysed is limited to those directly associated with that tumor or that group of tumors.

According to the invention, the panel of genes and the mutations to be analysed is chosen on the basis of the subject's anamnesis, obtained by a historic-family survey.

When the detected frequency of mutation shows a growth trend that exceeds a predefined value, for example 10%, (value which can be updated anyway, depending on the data that will be accumulated over the years) that is an increased number of the allelic frequency (according to what has been defined above) for a given mutation (for example, the allelic frequency of the mutation of the APC gene passes from of 0.1% to 5% in a subsequent test repetition), the monitoring system increases the sensitivity related to the panel being examined (i.e. it will ask the patient to carry out the test again, this time using a different panel having a greater sensitivity and analytical specificity with respect to the test of the first level) with regard to the genes involved in the increase of the mutations, up to 100%.

The method includes a continuous update of the evaluation parameters, such as the repetition frequency of the analysis cycles of the chosen panel of genes and the sensitivity related to the genes being analysed. In particular, the repetition frequency is defined on the basis of the stability index; more precisely, higher values of the instability index correspond to a greater repetition frequency of the tests, since it is assumed that an instability situation tends naturally to grow, and causes a higher probability to have new significant mutations more rapidly.

According to the invention, all the raw data and obtained results, related to the DNA analysed for each evaluation cycle, are recorded and processed during the subsequent cycles to improve the accuracy of evaluation as the quantity of the available data increases.

In order to make evaluation and prediction of the risk status as little invasive as possible, the biological sample taken from the individual to isolate the DNA to be analysed is constituted by a liquid biopsy (as already defined herein). In a preferred embodiment of the invention, the liquid biopsy is a peripheral blood sample.

In an embodiment of the invention, the liquid biopsy is urine.

The DNA isolated from the biological sample and used for the evaluation and prediction of the risk status is cfDNA (cell free DNA).

In an embodiment of the invention the cfDNA is isolated from plasma separated from the peripheral blood sample.

In another embodiment of the invention the cfDNA is isolated from plasma separated from the peripheral blood sample.

The method according to the invention includes also searching ctDNA (circulating tumor DNA) in the collected liquid biopsy.

When the ctDNA is detected, the method for evaluation and prediction of the risk substantially stops performing its task, since it means that at least one of mutation lines has caused the formation of neoplastic cells. At this point, the system for risk evaluation and prediction issues information aimed at transferring the control of the individual to an early detection system, such as, for example, the *SCED system - Solid Cancer Early Detection,* developed and used by the present Applicant.

## BRIEF DESCRIPTION OF THE DRAWINGS

The characteristics of the invention, as they become evident from the claims, are pointed out in the following detailed description, with reference to the enclosed tables of drawings, in which:

Fig. 1 illustrates a flow chart of the method for searching and identifying a prodromal genetic condition at the onset of solid tumors according to a general embodiment of the invention;

Fig. 2 illustrates a list of the panel of genes involved in the evaluation of the mutations which define a genetic stability or instability condition according to the method of the invention.

## DESCRIPTION OF PREFERRED EMBODIMENTS OF THE

## INVENTION

The present invention relates to a method for searching and identifying a genetic condition prodromal of the onset of solid tumors in a healthy individual, with the exception of brain tumors, for monitoring the individual over time in relation to possible entry in said condition, on the basis of the evolution of the trend of the individual's genetic stability.

Brain tumors are generally excluded from the approach used in the method for searching and identifying a genetic condition prodromal of the onset of solid tumors according to the invention. At present, scientific evidence in not enough to allow this approach to be used also for the brain tumors. The set of genes associated with the onset of this type of tumors has not been properly identified yet, and at present, they seem to be associated mainly with the DNA methylation state rather than with a specific mutation of its sequence. In fact, the proposed panels are not fit for evaluation of the DNA methylation state. Moreover, it is necessary to specify that, as already described by Bettegowda, C. et al. (Bettegowda, C. et al. Detection of circulating tumor DNA in early- and late-stage human malignancies. Science translational medicine 6, 224ra224, doi:10.1126/scitranslmed.3007094 - 2014), very likely the blood-brain barrier forms a filter that reduces considerably the presence of cfDNA in the general circulation. Therefore, the present techniques of extraction and subsequent analysis do not allow providing data strong enough to be used for an analysis of tumor risk.

The method includes carrying out, at predefined intervals, a series of periodical evaluation cycles of the mutation frequency that involve a panel of genes chosen from those associated to the onset of the cited solid tumors. Figure 1 illustrates, by way of example, a possible workflow of the steps of the method that will be described later on. Non fundamental changes of the workflow are possible without departing from the scope of the invention.

Unless otherwise stated, it is agreed that the technical terms used in the present treatment have a meaning commonly and unambiguously known to persons of ordinary skill in the field (for example, "liquid biopsy", "DNA isolation", "DNA amplification", "DNA sequencing", "ctDNA", "cfDNA", "Circulating Tumor Cells", etc.). It is also assumed that the techniques of molecular biology and genetic engineering, which are referred to and which are intended to be used for carrying out the method according to the invention (for example, *"NGS - Next Generation Sequencing"*), are standard techniques commonly used in practice and are also well known to the persons of ordinary skill in the field.

For each cycle of search and identification of a genetic condition prodromal of the onset of solid tumors, the method proposed by the invention includes isolating of the DNA from the biological sample which has been taken from an individual and then sequencing thereof, preferably with the NGS technique, after having suitably amplified the relevant fraction of DNA.

In order to make the evaluation and prediction of the risk status as little invasive as possible, the biological sample taken from the individual to isolate the DNA to analyse consists of a liquid biopsy. A liquid biopsy is substantially formed by a liquid or semi-liquid biological material circulating in the individual and produced by him/her by secretion or excretion substantially of a body fluid.

In a preferred embodiment of the invention, the liquid biopsy is a peripheral blood sample, which is taken and treated, if necessary, in order to separate the plasma or serum, depending on the subsequent use.

In a different embodiment of the invention, the liquid biopsy is urine.

According to the invention, the DNA isolated from the biological sample and used for the evaluation and prediction of the risk status is cfDNA (cell free DNA).

The existence of circulating free DNA, cfDNA (Cell Free DNA) has been demonstrated for the first time by Mendel and Metis about 70 years ago. The above mentioned DNA derives from the necrotic cells (premature death) and/or apoptotic cells (programmed death) and is generally released by all the types of cells. About 40 years after the discovery of the cf-DNA, Stroun *et al.* have demonstrated that specific carcinogenic alterations could be identified also in the cf-DNA. Afterwards, several articles have been published that confirm the existence of the circulating tumor DNA (ctDNA) by studying specific alterations associated with tumors. The ctDNA is thus a portion of the total cfDNA and has been estimated to represent 0.01% to 1% in very early stages up to 40% in the advanced stages, as already described by Bettegowda, C. et al. (Bettegowda, C. et al. Detection of circulating tumor DNA in early- and late-stage human malignancies. Science translational medicine 6, 224ra224, doi :10.11261scitranslmed.3007094 - 2014).

As already mentioned, in the blood, the apoptosis seems to be the most frequent process that generates cfDNA, although the portion provided by necrotic processes in cancer patients is not totally neglectable.

The quantity and variability of cfDNA in serum and plasma seems to be considerably greater in ill patients rather that in healthy controls, especially in cases of an advanced stage tumor rather than in early stages.

It is to be considered that the quantity of the cfDNA is influenced also by physiopathological conditions such as an inflammatory process, and since the cfDNA has a very low stability (from 15 minutes up to several hours), thus the reliability and coherence of the result are not always assured. In this case, an approach based on the periodical repetition of the test offers the advantage of obtaining a lower incidence of false negatives.

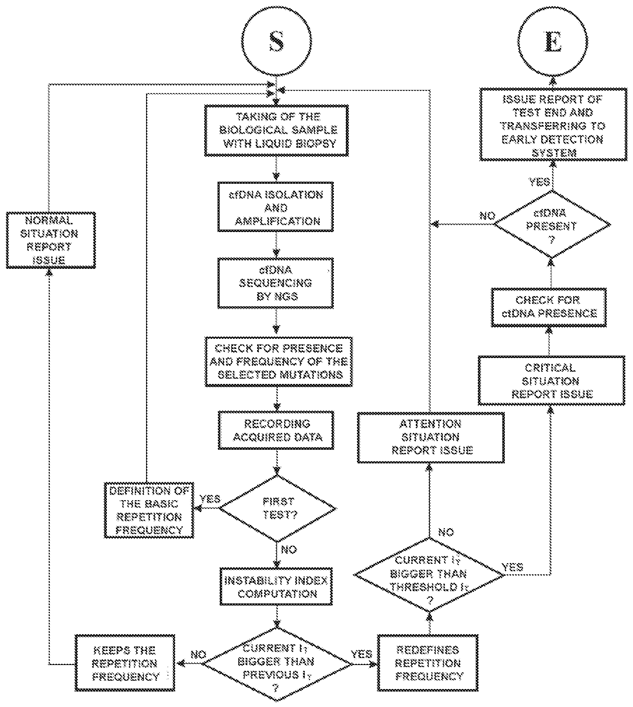

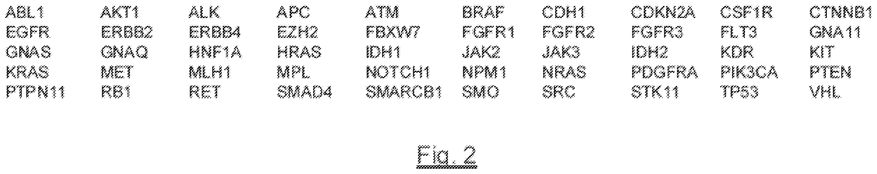

A study with an NGS panel, which can evaluate 50 genes involved in cancer (see Figure 2), covering 2,800 mutations (COSMIC) in 60 solid tumors and 31 blood tumors, has analysed 17 patients with breast metastatic tumor and a 76% concordance between tissue and plasma has been estimated. From these data the authors have drawn a conclusion that plasma can be considered the biological sample adopted for the screening of tumors as a substitute of the metastatic biopsy.

In an embodiment of the invention the cfDNA is isolated from plasma separated from the peripheral blood sample taken from the individual.

In a different embodiment of the invention the cfDNA is isolated from serum separated from the peripheral blood sample taken from the individual.

According to known techniques, the cfDNA present in the plasma can be isolated using magnetic beads covered with silica or silicone resins. In both cases, the capacity of DNA (negatively charged) to bind with silica (positively charged) in the presence of high concentrations of chaotropic salts having pH near 7.5, is exploited (Chen and Thomas, 1980; Marko et al. 1982; Boom et al. 1990). The binding of DNA to silica is induced by the dehydration induced by chaotropic salts and by the formation of hydrogen bonds which compete with weak electrostatic repulsions (Melzak *et al.* 1996). Afterwards, the exceeding salts, proteins, carbohydrates, metabolites and other contaminating substances are removed by repeated washing with alcoholic solutions. Finally, the purified DNA is eluted by a low ionic strength solution (like TE-buffer or water).

The next step of the method for evaluation and prediction of the risk according to the invention consists of the analysis of the chosen panel of genes (Figure 1), with particular attention to a chosen group of hotspots, which includes the evaluation of the presence of one or more mutations that involve the cited panel of genes, chosen from a list of known mutations for the monitored genes and indicative of an evolution toward the formation of neoplastic cells.

For example, about 10% of the patients suffering from lung tumor (non small cell type) in the United States and Europe are characterized, from the genetic point of view, by the mutations of the EGFR gene (Lynch et al 2004 ;. Paez et al 2004 ;. Pao *et al.,* 2004). These mutations take place mainly within the EGFR exons 18-21, which codify a portion of the tyrosine kinase domain of EGFR. About 90% of these mutations involving the exon 19, are delections or in the exon 21 are SNV (like the mutation L858R) (Ladanyi and Pao 2008). These mutations increase the tyrosine kinase activity of the EGFR protein, which determines a hyper-activation of the cellular pathways that stimulate the survival of the tumor cells (Sordella et al. 2004). Regardless of the ethnic origin, mutations of the EGFR gene are more frequently found in non smoking female patients (less than 100 cigarettes during the patient's whole life) with adenocarcinoma type histology (Lynch *et al.* 2004). However, these mutations that involve the EGFR gene can be found also in other subgroups of patients with lung carcinoma, thus also in smokers. The presence of the above mentioned mutations in the cfDNA of a seemingly "healthy" patient represents an obvious alarm bell which must be necessarily followed by a monitoring and a series of thorough exams to evaluate the presence of a tumor mass.

In particular, cfDNA is evaluated with NGS techniques (next-generation sequencing) on 50 genes, a list of which is provided in figure 2, which covers currently 2,800 COSMIC mutations (Catalogue Of Somatic Mutations In Cancer).

It will be appreciated that, for the purposes of the invention, both the list of genes and the list of the mutations which are evaluated, must not be considered static, since the intensive research activity in this field as well as the available modern sequencing and analysis techniques can lead to the identification of new genes, new hotspots or new mutations involved in the carcinogenesis of one or more solid tumor forms.

Each evaluation cycle searches, for each monitored gene, the mutations involving it (see the above example of the EGFR), with particular attention to mutations that occur in known hotspots. When a significant mutation is present, this is recorded in a memory area of a computerized system which is physically appointed to carry out computational steps of the method (figure 1).

In each repetition cycle of the test, that is in each cycle after the first one, it is checked whether the detected mutation has already been detected in the previous evaluation cycles. In a negative case, the new mutation involving this gene is registered and an algorithm calculates a value indicative of the trend that the frequency of mutation shows over time. This value constitutes the "Key Risk Indicator" of the system.

The trend is represented by a numerical value, obtained by the computation of a relation between the last frequency value of the mutation and the values obtained in the previous evaluation cycles, and its tendency can be represented in a diagram to provide indications of the level of the genetic stability or instability.

In a non-limiting embodiment of the method according to the invention, the comparison between the two checks can be summed up by an overall genetic instability index $I_T$ according to the formula:

$$I_T = \sum_{k=1}^{n} \frac{\Delta F_k}{n}$$

Where:

 $n$: number of genes on which the control is performed
 $F_k$: mutation frequency of a single gene

It is to be noted that in this case the overall instability index $I_T$ expresses the overall situation, without distinguishing between the variations on the specific genes.

In a similar way, it is possible to define an instability index $I_G$ of a specific gene according to the formula:

$$I_G = \sum_{j=1}^{n} \frac{\Delta F_j}{n}$$

Where:

 $n$: number of evaluated hotspots of the gene on which the control is performed
 $F_j$: mutation frequency of a single hotspot in the specific gene

The panels of genes and their mutations taken into consideration for every evaluation cycle can include again the whole range of 50 genes associated with the onset of solid tumors (figure 2), or only some genes and hotspots associated only with one or more chosen tumors.

In particular, it is possible to monitor the risk of the onset of only one type of tumor, or a single family of tumors. In this case, the number of genes being analysed is limited to those directly associated with that tumor or that group of tumors.

In any case, the definition of the instability index according to the above described process can involve, depending on the prefixed search target, a single gene, a panel consisting of a set of genes associated to the onset of a specific tumor or family of tumors, or the whole panel consisting of the 50 genes (at present, or more genes, if, in future, other genes are identified) associated with the onset of solid tumors in general.

According to the invention, the panel of genes and the mutations to be analysed is chosen on the basis of the subject's anamnesis, obtained by a historic-family survey.

When an evaluation cycle detects a frequency of mutation that expresses a growth trend higher than 10%, (value which can be updated depending on the data that will be accumulated over the years) that is an increased number of the allelic frequency for a determined mutation (for example, the allelic frequency of the mutation of the APC gene passes from of 0.1% to 5% in a subsequent test repetition), the monitoring system increases the sensitivity related to the panel being examined (i.e. it will ask the patient to carry out the test again, this time using a different panel having a greater sensitivity and analytical specificity with respect to the test of the first level) with regard to the genes involved in the increase of the mutations, up to 100%.

For example, using the panels and commercial technology "Oncomine® cfDNA", extremely low detection levels are reached, equal to 0.05%. It means that the system, based on a particular chemistry ("Oncomine® TagSequencing"), different from the screening of the first level, is capable of identifying a mutation present in barely 0.05% of the analysed DNA sample.

The method includes a continuous update of the evaluation parameters, such as the repetition frequency of the analysis cycles of a chosen panel of genes and the sensitivity related to the genes being analysed. In particular, the repetition frequency is defined on the basis of the instability index ($I_T$ or $I_G$, depending on whether a panel of several genes or a single gene is analysed); more precisely, low values of the instability index correspond to a basic repetition frequency of the tests, which can be for example of one test per year. Even moderate increase of such index value can suggest an increased repetition frequency, since it is considered that an instability situation tends naturally to increase and implies a bigger probability of new significant mutations in shorter time. The exceeding of a specific threshold value $I_{TS}$, $I_{GS}$ of the instability index $I_T$, $I_G$ identifies an evolution of the sequence of mutations in a prodromal genetic condition at the onset of the tumor or group of tumors being monitored, that is in a path which will lead the individual, certainly or very likely, to develop the above mentioned tumor, or in any case, at least one of the monitored tumors. The threshold value of the instability index can be for example set to 0.1.

According to the invention, gradual increase of the repetition frequency of the tests allows best monitorinf of the individual's situation and understanding when the specific mutations can occur, indicative of an oncogenesis underway.

According to the invention, all the raw data and obtained results, related to the DNA analysed for each evaluation cycle, are recorded and revised during the subsequent cycles to improve the accuracy of evaluation as the quantity of the available data increases.

According to another characteristic of the method of the invention, the sequencing of the cfDNA for the study of the somatic mutations is combined with the analysis of the germ mutations. For this purpose, according to what has been already described, the cfDNA with the Hotspot Cancer Panel (HSCP) is isolated and sequenced, which allows to study 2800 mutations in 50 genes involved in neoplastic processes with a 1% sensitivity.

Moreover, the individual's germ DNA, for example lymphocyte DNA, is isolated and sequenced, and the presence of the same mutations in the above mentioned DNA is checked. For the purposes of the invention, the individual's lymphocyte DNA can derive indifferently from the same liquid biopsy from which the cfDNA has been taken and isolated, or from another, recent or even much older biopsy, since the germ mutations present in the lymphocyte DNA form part of the individual's genetic heritage.

The lymphocyte DNA is sequenced preferably with the same reading degree of the cfDNA sequencing to obtain directly comparable results.

A mutation is thus defined somatic, if it is present in the cfDNA analysis and not in the lymphocyte DNA sequenced with the same reading degree. Thus the following sets of mutations are defined:

Set A: consisting of mutations found in the cfDNA;
Set B: consisting of mutations found in the lymphocyte DNA analyzed by the same panel used for the cfDNA;
Set C: consisting of somatic mutations defined as the mutations present in the cfDNA, but not in the lymphocytes.

When a somatic mutation is identified (that is, in the analysis, the set C is different from empty) the tissues in which such a somatic mutation has been mostly found, are evaluated with known operational techniques, by the COSMIC database.

The next step is to study if there is a higher probability to develop a tumor of these tissues, from the analysis of the germ line, using operational techniques, known also in this case. The above mentioned approach will be carried out by a customized panel, whose definition is a function of the found somatic mutation or somatic mutations, and allows to evaluate the individual's susceptibility to particular tumors on the basis of the mutated gene (or the mutated genes) in the cfDNA.

Furthermore, if the somatic mutation concerned involves the lung, colon or breast, the circulating DNA is analysed with a higher sensitivity (up to 0.1%), for example by means of commercial panels Oncomine®, and using the commercial technology Tag_Seq®.

When these first analyses are completed, the evaluation is finished proposing an oncological consultation to explain and evaluate the results and to plan new tests in order to monitor the individual being examined.

The method according to the invention includes also searching the ctDNA (circulating tumor DNA) in the taken liquid biopsy, as an additional activity that completes the evaluation of the genetic stability and identification of the prodromal phase of the oncogenesis. Such operation can be performed only when the calculated instability index exceeds the prefixed threshold value $I_{TS}$, $I_{GS}$, as indicated in figure 1, or also when the values of the instability index are below this threshold, as a precaution.

When the ctDNA is detected, the method for evaluation of the genetic stability and identification of the prodromal phase of the oncogenesis substantially ends its function, since it means that at least one of the mutation lines has caused the formation of neoplastic cells. At this point, the system for evaluation of the genetic condition gives information to transfer the control of the individual to an early detection system, ("*Early Detection*"), such as for example the *"SCED system - Solid Cancer Early Detection",* developed and used by the present Applicant.

Some applications will be described in the following by way of example, focused on the evaluation of the genetic stability and identification of the prodromal phase of the oncogenesis related to single solid tumors or families of solid tumors, and in particular to lung, breast and ovarian, and colorectal tumors.

Example 1: Lung tumors

In an embodiment of the invention, the method for the evaluation of the genetic stability and identification of the prodromal phase of the oncogenesis is applied to monitoring of the genetic condition associated with the onset of the lung tumors.

The most serious risk factor for the onset of a lung tumor is represented by cigarette smoking. A clear correspondence between the amount of smoke inhaled by a smoker and the increase of the probability to contract such tumor has been widely proved and is already considered a fact.

Several studies report that the risk to contract a lung tumor is 14 times higher for smokers than non smokers (up to 20 times for heavy smokers - more than 20 cigarettes a day). The cigarette smoke is responsible for 8/9 out of every 10 lung tumors, though atmospheric pollution, family predisposition for this type of cancer, and the presence of other lung diseases may increase the likelihood of contracting a tumor.

On the basis of the quantification of his/her personal risk that specific mutations of particular genes associated with lung tumors and the number and frequency of such mutations can generate tumor cells in future, the person being monitored is offered the possibility of knowing, with adequate reliability, whether a development is detected and which evolution stage has been reached.

According to the present method, the definition of the prodromal state of the tumor onset in this case is connected to the mutation of 11 genes involved directly in lung tumors, in particular of 169 different hotspots. Table 1 provides a list of genes and hotspots that compose the panel, which will be evaluated.

An application of the method for the evaluation of the genetic instability with regard to the panel related to lung tumors for a healthy individual is described by way of example.

10 cc of peripheral blood are taken from a 45 years old male patient; the blood is centrifuged so as to separate the plasma (containing the circulating free DNA) from the corpuscular component (lymphocytes and erythrocytes). In this patient, 14 uL of cfDNA are extracted at a concentration of 2.36 ng/uL, starting from 4 ml of plasma. 20 ng of cfDNA are used to make what is called "NGS library" that is a set of DNA fragments which are associated to a barcode (a synthetic DNA sequence) that defines the specimen in an unambiguous manner. On the basis of the read concentration (3390 pM), such a library is mixed (pooling) with the libraries obtained from other specimens (each of which will have a different barcode). The cfDNA is sequenced and the mutations in the panel of genes and hotspots of Table 1 (lung) are analysed; the mutation p.G12D of the KRAS gene at 0.49% is found.

After 6 months the same analysis is repeated and the same mutation p.G12D of the KRAS gene at 1.05% is found; the instability index is calculated with the formula

$$l_G = \sum_{j=1}^{n} \frac{\Delta F_j}{n}$$

and the value 0,047 is obtained. Since the index value is below the 0.1 threshold it is recommended to repeat the test after 6 months.

Table 1

| | LUNG cfDNA HOTSPOTS |
|---|---|
| **Gene** | **Amino-acid change** |
| NRAS | p.Q61L p.Q61K, pA59T, p.G13V, p.G13D, pG13Y, p.G13V, p.G13A, p.G13N, p.G13R, p.G13C, p.G13S, p.G12E, p.G12D, p.G12P, p.G12Y, p.G12A, p.G12V, p.G12N, p.G12R, p.G12C, p.G12S |
| ALK | p.R1275L, p.R1275Q, p.F1245L, p.F1245L, p.F1245C, p.F1245I, p.F1245V, p.L1196Q, p.L1196M, p.V1180L, p.F1174L, p.F1174L, p.F1174C, p.F1174S, p.F1174I, p.F1174V, p.F1174L, p.I1171N, pT1151_L1152insT, p.G1128A |
| PIK3CA | p.E542K, p.E545K, p.H1047R |
| ROS1 | p.L1951M |

(continued)

| | LUNG cfDNA HOTSPOTS |
|---|---|
| Gene | Amino-acid change |
| EGFR | p.E709K, p.E709A, p.G719C, p.G719C, p.G719A, p.K745_E746insIPVAIK, p.E746_A750delEREA, p.E746_A750delELREA, p.E746_T751A, p.E746_S752V, p.L747_E749delILRE, p.L747_A750P, p.L747_T751P, p.L747_S752delILREATS, p.L747_T751delILREAT, p.L747_P753S, p.S768I, p.V769_S770insASV, p. D770_N771insSVD, p.H773_V774insH, p.H773_V774insNPH, p.T790M, p.C797S, p.E709_T710>D, p.E709_T710>A, p.E709_T710>G, p.E709H, p.E709G, p. E709V, p.G719D, p.H835L, p.P848L, p.L858R, p.L861Q |
| MET | p.T1010I, p.Y1021N, p.Y1021F, p.L982_D1028del, p.L982_D1028del, X1010_splice, p.H1112Y, p.H1112L, p.H1112R, p.Y1248H, p.Y1248C, p.Y1253N, p.Y1253H, p.Y1253D, p.M1268V, p.M1268T, p.M1268I |
| BRAF | p.V600E, p.G469V, p.G466V, p.Y472C, p.L597V, p.G469A, p.G469L |
| KRAS | p.Q61H, p.Q61R, p.Q61L, p.G13D, p.G13C, p.G12V, p.G12D, p.G12A, p.G12F, p.G12C, p.G12S, p.G12R |
| MAP2K1 | p.F53I, p.F53L, p.F53L, p.F53L, p.K57Q, p.Q56P, p.K57T, p.K57N, p.P124S, p.P124Q, p.124L, p.E203K, p.E203V |
| TP53 | p.R337L, p.R283P, p.R282W, p.R280I, p.C277F, p.R273H, p.R273L, p.R273P, p.273C, p.R249S, p.R249S, p.R249M, p.R248Q, p.R248L, p.R248W, p.G245V, p.G245C, p.C242F, p.M237I, p.Y234C, p.Y220C, p.H214R, p.Y205C, p.H179R, p.C176F, p.C176Y, p.R175H, p.V173L, p.Y163C, p.A159V, p.R158L, p.V157F, p.G154V, p.T125T |
| ERBB2 | p.A775_G776insYVMA, |

## Example 2: Breast and ovarian tumors

The test for evaluation of the genetic stability and identification of the prodromal phase of the oncogenesis which carries out the method according to the invention is applied in a particular way to women who undergo, or have undergone in the past, hormone replacement therapies, contraception, or ovarian stimulation.

Moreover, it can be advantageously used in other specific cases of monitoring and prevention, for example prevention program for women who carry hereditary BRCA 1|2 mutation, with high risk to develop the uterine or ovarian tumor.

The panel of genes and mutations used in this case includes 10 genes and 159 hotspot, listed in the table (see Table 2).

An application of the method for evaluation of the genetic instability with regard to the panel related to breast-ovarian tumors for a healthy individual is described by way of example.

10 cc of peripheral blood are taken from a 57 years old female patient; the blood is centrifuged so as to separate the plasma (containing the circulating free DNA) from the corpuscular component (lymphocytes and erythrocytes). In this patient, 14 uL of cfDNA are extracted at a concentration of 1.71 ng/uL, starting from 4 ml of plasma. 20 ng of cfDNA are used to make what is called "NGS library" that is a set of DNA fragments which are associated to a barcode (a synthetic DNA sequence) that defines the specimen in an unambiguous manner. On the basis of the read concentration (3240 pM), such a library is mixed (pooling) with the libraries obtained from other specimens (each of which will have a different barcode). The cfDNA is sequenced and the mutations in the panel of genes and hotspots of Table 2 (breast and ovaries) are analysed; the mutation p.H1047R of the PIK3CA gene at 0.57% is found.

After 6 months the same analysis is repeated and the mutations p.H1047R of the PIK3CA gene at 1.75 % are found and a new mutation p.R175H of the TP53 gene at 0.34% is found; the instability index is calculated with the formula

$$I_T = \sum_{k=1}^{n} \frac{\Delta F_k}{n}$$

and the value 0.152 is obtained. Since the index value is over the 0.1 threshold, it is recommended to repeat the test after 3 months.

Table 2

**BREAST cfDNA HOTSPOTS**

| Gene | Amino-acid change |
|---|---|
| SF3B1 | p.K700E p.N345K, p.C420R, p.E453K, p.E542K, p.E545Q, p.E545K, p.E545A, p.545G, p.Q546K, p.Q546R, p.Q546P, p.E726K, p.M1043V, p.M1043I, p.H1047Y, p.H.1047R, p.H1047L |
| PIK3CA | p.G1049R |
| FBXW7 | p.D600Y, p.S582L |
| ESR1 | p.E380Q, p.V392I, p.S463P, p.Y537N, p.Y537C, p.Y537S, p.D.538G |
| EGFR | p.H835L, p.P848L, p.L858R, p.861Q |
| KRAS | p.G13D, p.G13C, p.G12A, p.G12D, p.G12D, p.G12F, p.G12V, p.G12R, p.G12C, p.G12S |
| AKT1 | p.E286G, p.E286K, p.E285K, p.R283P, p.R282G, p.R282W, p.R280I, p.R280K, p.R280T, p.G279E, p.P278R, p.P278L, p.P278A, p.P278S, p.P278T, |
| TP53 | p.C277F, p.C275Y, p.V274L, p.V274F, p.R273H, p.R273L, p.R273P, p.R273C, p.V272L, p.V272M, p.G266M, p.G266V, p.G266R, p.G262V, p.E258K, p.P250L, p.R249S, p.R249K, p.R249M, p.R248Q, p.R248L, p.R248W, p.M246V, p.G245D, p.G245V, p.G245C, p.G245S, p.G244D, p.G244V, p.G244C, p.G244S, p.C242F, p.C242Y, p.S241F, p.S240G, p.C238F, p.C238Y, p.M237I, p.Y234C, p.Y220C, p.Y220H, p.V216M, p.H214R, p.R213Q, p.R213L, p.V197M, p.I195T, p.L194R, p.H193R, p.H193Y, p.P191del, p.P190L, p.P177_C182del, p.H179R, p.H179L, p.H179Y, p.C176F, p.C176Y, p.R175H, p.R175L, p.R175C, p.V173L, p.V173M, p.V172F, p.R158H, p.R158L, p.V157F, p.R156P, p.G154V, p.P152L, p.P151H, p.P151S, p.P151T, p.L145P, p.C141Y, p.141R, p.A138V, p.C135W, p.C135F, p.C135Y, p.K132R, p.K132E |
| ERBB2 | p.L755M, p.L755S |
| ERBB3 | p.R103G, p.V104M, p.V104M, p.V104M, p.V104L, p.V104L, p.V104L, p.G284R, p.G284R, p.G284R p.G284R, p.D297Y, p.D297Y, p.T355I, p.T355I, p.E928G |

Example 3: Colon and rectal tumors

The evaluation of the genetic stability and identification of the prodromal phase of the oncogenesis related to the category of colon and rectal tumors includes the periodical analysis of 14 genes and 246 hotspots, as specified in Table 3, which lists all the genes currently involved with the respective hotspots.

The neoplasia that involves the colorectal system often develops as the evolution of a benign lesion, such as adenomatous polyposis, in the intestinal mucous membrane.

The formation of neoplasia can be fostered by some risk factors, like obesity or a diet rich in calories and fats and low in fiber, or genetic factors, for example, a family history of the pathology. Moreover, the age, chronic intestinal inflammatory pathologies and medical history of polyps can likewise contribute and increase the probability of the onset of the tumor.

The time it takes for a benign neoplasm to become malignant is very often long (7 to 15 years), and such evolution can be advantageously followed with the application of periodical tests and the consequent evaluation of the risk status provided by the method according to the invention.

An application of the method for evaluation of the genetic instability with regard to the panel related to colorectal tumors for a healthy individual is described by way of example.

10 cc of peripheral blood are taken from a 65 years old male patient; the blood is centrifuged so as to separate the plasma (containing the circulating free DNA) from the corpuscular component (lymphocytes and erythrocytes). In this patient, 14 uL of cfDNA are extracted at a concentration of 1.49 ng/uL, starting from 4 ml of plasma. 20 ng of cfDNA are used to make what is called "NGS library" that is a set of DNA fragments which are associated to a barcode (a synthetic DNA sequence) that defines the specimen in an unambiguous manner. On the basis of the read concentration (8130 pM), such a library is mixed (pooling) with the libraries obtained from other specimens (each of which will have a different barcode). The cfDNA is sequenced and the mutations in the panel of genes and hotspots of Table 1 (lung) are analysed; a mutation p.R1450Ter of the APC gene at 0.15% is found.

After 6 months the same analysis is repeated and the same mutation p.R1450* of the APC gene at 1.05% is found; the instability index is calculated with the formula

$$I_G = \sum_{j=1}^{n} \frac{\Delta F_j}{n}$$

and the value 0.026 is obtained. Since the index value is below the 0.1 threshold, it is recommended to repeat the test after 6 months.

Table 3

|  | COLON cfDNA HOTSPOTS |
|---|---|
| Gene | Amino-acid change |
| NRAS | p.Q61L, p.Q61R, p.Q61K, p.G13V, p.G13V, p.G13A, p.G13D, p.G13Y, p.G13N, p.G13S, p.G13R, p.G13C, p.G12E, p.G12V, p.G12D, p.G12A, p.G12P, p.G12Y, p.G12N, p.G12S, p.G12C, p.G12R |
| CTNNB1 | p.S33Y, p.G34V, p.T41A, p.T411, p.S45P, p.S45F |
| PIK3CA | p.E542K, p.E545Q, p.E545K, p.E545A, p.E545G, p.Q546K, p.Q546R, p.Q546P, p.M1043V, p.M1043I, p.H1047Y, p.H1047R, p.H1047L, p.G1049R |
| FBXW7 | p.R689W, p.D600Y, p.S582L, p.W526R, p.R505C, p.R479Q, p.R465H, p.R465C |
| APC | p.R805Ter, p.R876Ter, p.Y935Ter, p.R1114Ter, p.S1234fs, p.Q1291Ter, p.Q1294Ter, p.Q1303Ter, p.E1306Ter, p.I1307fs, p.E1309fs, p.E1309fs, p.E1309Ter, p.E1309fs, p.E1309fs, p.G1312Ter, p.E1353Ter, p.P1361fs, p.Q1367Ter, p.P1372fs, p.P1373fs, p.Q1378Ter, p.E1379Ter, p.Q1406Ter, p.E1408Ter, p.S1411fs, p.R1450Ter, p.S1465fs, p.E1464fs, p.S1465fs, p.L1488fs, p.F1491fs, p.T1493fs, p.T1556fs, p.E1577Ter |
| EGFR | p.R51C, p.S464L, p.G465R, p.G465R, p.G465R, p.G465E, p.K476T, p.I491M, p.S492R, p.S492R |
| BRAF | p.V600E, p.L597V, p.D594G |
| KRAS | p.A146T, p.Q61H, p.Q61R, p.Q61L, p.G13D, p.G13C, p.G12A, p.G12D, p.G12V, p.G12F, p.G12R, p.G12C, p.G12S |

(continued)

|  | COLON cfDNA HOTSPOTS |
|---|---|
| Gene | Amino-acid change |
| AKT1 | p.E17K |
| MAP2K1 | p.F53I, p.F53L, p.F53C, p.F53L, p.Q56P, p.K57Q, p.K57T, p.K57N, p.E203K, p.E203V |
| TP53 | p.E286G, p.E286K, p.E285K, p.R283P, p.R282W, p.R282G, p.280I, p.R280K, p.R280T, p.G279E, p.P278R, p.P278L, p.P278A, p.P278S, p.P278T, p.C277F, p.C275Y, p.V274L, p.V274F, p.R273H, p.R273L, p.R273P, p.R273C, p.V272L, p.V272M, p.G266E, p.G266V, p.G266R, p.G262V, p.E258K, p.P250L, p.R249S, p.R249K, p.R249M, p.R248Q, p.R248L, p.R248W, p.M246V, p.G245D, p.G245V, p.G245S, p.G245C, p.G244D, p.G244V, p.G244C, p.G244S, p.C242F, p.C242Y, p.S241F, p.S240G, p.C238F, p.C238Y, p.M237I, p.Y234C, p.Y220C, p.Y220H, p.V216M, p.H214R, p.R213Q, p.R213L, p.V197M, p.I195T, p.L194R, p.H193R, p.H193Y, p.P191del, p.P190L, p.P177_C182del, p.H179R, p.H179L, p.H179Y, p.C176F, p.C176Y, p.R175H, p.R175L, p.R175C, p.V173L, p.V173M p.V172F, p.R158L, p.R158H, p.V157F, p.R156P, p.G154V, p.P152L, p.P151H, p.P151S, p.P151T, p.L145P, p.C141Y, p.C141R, p.A138V, p.C135W, p.C135F, p.C135Y, p.K132R, p.K132E |
| ERBB2 | p.S310F, p.S310Y, p.L755M, p.L755S, p.E770_A771insAYVM, p.G776V, p.V777L, p.V842I, p.R896CS |
| SMAD4 | p.A118V, p.E330A, p.D351G, p.P356L, p.R361C, p.R361H, p.G386D, p.G510V |
| GNAS | p.R201C, p.R201S, p.R201H, p.R201L, p.0227R |

It is understood that what above has been described as a pure not limiting example. Therefore, possible changes and variants of the invention are considered within the protective scope granted to the present method, as described above and claimed below.

## References

- Cancer Genome Landscapes Science. 2013 Mar 29; 339(6127): 1546-1558.. Bert Vogelstein, Nickolas Papadopoulos, Victor E. Velculescu, Shibin Zhou, Luis A. Diaz, Jr., and Kenneth W. Kinzler*

- Hereditary Cancer Risk Assessment: New Perspectives and Challenges for the Next-Gen Sequencing Era. Front Oncol. 2016; 6: 133. Israel Gomy

- Principles in genetic risk assessment Ther Clin Risk Manag. 2005 Mar; 1(1): 15-20. Pedro Viana Baptista

- Predictive genomics: A cancer hallmark network framework for predicting tumor clinical phenotypes using genome sequencing data. Wang E. et al.

- Mandel P, Metais P. Les acides nucleiques du plasma sanguin chez l' homme [The nucleic acids in blood plasma in humans]. C R Seances Soc Biol Fil. 1948;142(3-4):241-243.

- Stroun M, Anker P, Maurice P, Lyautey J, Lederrey C, Beljanski M. Neoplastic characteristics of the DNA found in the plasma of cancer patients. Oncology. 1989;46(5):318-322

- Sausen M, Phallen J, Adleff V, Jones S, Leary RJ, Barrett MT, Anagnostou V, Parpart-Li S, Murphy D, Kay Li Q, Hruban CA, Scharpf R, White JR, O'Dwyer PJ, Allen PJ, Eshleman JR, Thompson CB, Klimstra DS, Linehan DC, Maitra A, Hruban RH, Diaz LA Jr, Von Hoff DD, Johansen JS, Drebin JA, Velculescu VE. Clinical implications of genomic alterations in the tumour and circulation of pancreatic cancer patients. Nat Commun. 2015 Jul 7;6:7686. doi: 10.1038/ncomms8686.

- Hao TB, Shi W, Shen XJ, et al. Circulating cell-free DNA in serum as a biomarker for diagnosis and prognostic prediction of colorectal cancer. Br J Cancer. 2014;111(8):1482-1489.

- Zonta E, Nizard P, Taly V. Assessment of DNA integrity, applications for cancer research. Adv Clin Chem.

2015;70:197-246

- Chen, C.W. and Thomas, C.A. Jr. (1980) Recovery of DNA segments from agarose gels. Anal. Biochem.101, 339-41.

- Marko, M.A. et al. (1982) A procedure for the large-scale isolation of highly purified plasmid DNA using alkaline extraction and binding to glass powder. Anal. Biochem. 121, 382-7.

- Boom, R. et al. (1990) Rapid and simple method for purification of nucleic acids. J. Clin. Microbiol. 28, 495-503.

- Melzak, K.A. et al. (1996) Driving forces for DNA adsorption to silica in perchlorate solutions. J. Colloid Interface Sci. (USA) 181, 635-44.

Jones S, Anagnostou V, Lytle K, Parpart-Li S, Nesselbush M, Riley DR, Shukla M, Chesnick B, Kadan M, Papp E et al: Personalized genomic analyses for cancer mutation discovery and interpretation. Science translational medicine 2015, 7(283):283ra253.

Ng CK, Piscuoglio S, Geyer FC, Burke KA, Pareja F, Eberle C, Lim R, Natrajan R, Riaz N, Mariani O et al: The Landscape of Somatic Genetic Alterations in Metaplastic Breast Carcinomas. Clinical cancer research : an official journal of the American Association for Cancer Research 2017.

Gagan J, Van Allen EM: Next-generation sequencing to guide cancer therapy. Genome medicine 2015, 7(1):80.

Genomes Project C, Auton A, Brooks LD, Durbin RM, Garrison EP, Kang HM, Korbel JO, Marchini JL, McCarthy S, McVean GA et al: A global reference for human genetic variation. Nature 2015, 526(7571):68-74.

Ashworth A, Lord CJ, Reis-Filho JS: Genetic interactions in cancer progression and treatment. Cell 2011, 145(1):30-38.

Richards S, Aziz N, Bale S, Bick D, Das S, Gastier-Foster J, Grody WW, Hegde M, Lyon E, Spector E et al: Standards and guidelines for the interpretation of sequence variants: a joint consensus recommendation of the American College of Medical Genetics and Genomics and the Association for Molecular Pathology. Genetics in medicine : official journal of the American College of Medical Genetics 2015, 17(5):405-424.

7.De Mattos-Arruda L, Caldas C: Cell-free circulating tumour DNA as a liquid biopsy in breast cancer. Molecular oncology 2016, 10(3):464-474.

8.Diaz LA, Jr., Bardelli A: Liquid biopsies: genotyping circulating tumor DNA. Journal of clinical oncology : official journal of the American Society of Clinical Oncology 2014, 32(6):579-586.

9.Pantel K, Diaz LA, Jr., Polyak K: Tracking tumor resistance using 'liquid biopsies'. Nature medicine 2013, 19(6):676-677.

10.Garraway LA, Verweij J, Ballman KV: Precision oncology: an overview. Journal of clinical oncology : official journal of the American Society of Clinical Oncology 2013, 31(15):1803-1805.

11.Yohe SL, Carter AB, Pfeifer JD, Crawford JM, Cushman-Vokoun A, Caughron S, Leonard DG: Standards for Clinical Grade Genomic Databases. Archives of pathology & laboratory medicine 2015, 139(11):1400-1412.

12.Aaboud M, Aad G, Abbott B, Abdallah J, Abdinov O, Abeloos B, Aben R, AbouZeid OS, Abraham NL, Abramowicz H et al: Search for triboson [Formula: see text] production in pp collisions at [Formula: see text] [Formula: see text] with the ATLAS detector. The European physical journal C, Particles and fields 2017, 77(3):141.

13.Dacheva D, Dodova R, Popov I, Goranova T, Mitkova A, Mitev V, Kaneva R: Validation of an NGS Approach for Diagnostic BRCA1/BRCA2 Mutation Testing. Molecular diagnosis & therapy 2015, 19(2):119-130.

Bettegowda C, Sausen M, Leary RJ, Kinde I, Wang Y, Agrawal N, Bartlett BR, Wang H, Luber B, Alani RM et al: Detection of circulating tumor DNA in early- and late-stage human malignancies. Science translational medicine 2014, 6(224):224ra224.

**Claims**

1. A method for searching and identifying a genetic condition prodromal of the onset of solid tumors in a healthy subject, **characterized by** including an evaluation cycle for the evaluation of genetic stability or instability condition and at least one repetition cycle of such evaluation, said repetition cycles being periodically performed on the subject, with each cycle comprising the steps of:

   - isolating DNA from the biological material of a sample which has been taken from the subject, amplifying and sequencing the isolated DNA;
   - verifying the presence of mutations selected in a predetermined set of genes of the sample under consideration, said set of genes and said mutations being associated with the onset of solid tumors;
   - the predetermined set of genes and selected mutations being defined in view of the subject's anamnesis obtained by a historic-family survey;
   - the predetermined set of genes including either a subset of a panel of genes or hotspots, consisting of known positions in genes that frequently mutate in cancer patients, and that are connected to one or more solid tumors, or the entire set of genes connected to solid tumors;
   - verifying the frequency of mutations detected for each gene and for each evaluation cycle, the mutations being chosen from the aforementioned selected mutations;
   - recording the mutations detected for each gene or group of genes and their frequency;
   - defining or updating a genetic instability index of the subject, either overall ($I_T$,) or for a single gene ($I_G$), for each repetition cycle, based on the frequency of mutations detected, said genetic instability index ($I_T$, $I_G$) being also defined on the basis of the increase in the frequency of mutations with respect to one or more previous evaluation cycles, the value of said overall instability index (It) being a function of the average of the mutation frequency variations of all genes, and the value of said instability index for each individual gene being a function of the average of the mutation frequency variations of each individual hotspot of the considered gene;
   - evaluating, in each repetition cycle, the subject's entry into a genetic condition prodromal of the onset of one or more solid tumors or groups of solid tumors on the basis of a threshold value ($I_{TS}$,$I_{GS}$) of said genetic instability index ($I_T$,$I_G$), defined for each single gene or group of genes, being exceeded.

2. The method of claim 1, wherein said overall genetic instability index ($I_T$) is defined as the summation of the relationships between a value ($\Delta F_k$) responsive to the increase in the observed mutations for each gene and the number of genes evaluated in consideration of the whole group of monitored genes.

3. The method of claim 1, wherein said index of genetic instability for single gene ($I_G$) is defined as the summation of the relationships between a value ($\Delta F_j$) responsive to the increase in the observed mutations for each hotspot and the number of hotspots evaluated, in consideration of the whole group of monitored hotspots.

4. The method of claim 1, wherein the biological sample consists of a liquid biopsy, and the phase of verification of the presence of mutations in the predetermined set of genes is performed on a DNA sample isolated from said liquid biopsy and subsequently amplified and sequenced.

5. The method of claim 4, wherein the liquid biopsy is peripheral blood.

6. The method of claim 4, wherein the liquid biopsy is urine or spinal fluid.

7. A method according to one of the claims 4 to 6, wherein a fraction of cfDNA is sought in the DNA sample being analyzed and wherein the presence of mutations is verified in said cfDNA fraction.

8. A method according to one of the claims 4 to 6, wherein the ctDNA isolated from the liquid biopsy is also sought in the DNA sample being analyzed.

9. A method of claim 8, wherein, following the identification of circulating ctDNA, addressing information is sent to an early detection system ("*Early Detection*").

10. The method of claim 8, wherein, following the identification of ctDNA in said DNA sample being analyzed, the presence of CTC ("*Circulating Tumor Cells*") is sought in said liquid biopsy.

11. The method of claim 1, wherein the predetermined set of genes and selected mutations are defined in view of their connection to particular types of tumor.

12. A method of claim 1, wherein the repetition period is recalculated after each repetition cycle according to a value of said instability index ($I_T,I_G$) calculated in the current cycle and a value of the same as calculated in one or more previous cycles.

13. A method of claim 1, wherein a greater analysis sensitivity related to the monitored gene or panel of genes is set in each repetition cycle, following an increase in the calculated value for said instability index ($I_T,I_G$) with respect to the value of the same index ($I_T$, $I_G$) calculated in one or more previous cycles.

14. The method of claim 1, wherein, in each of said evaluation and repetition cycles, the germ DNA is also isolated and sequenced, and only the mutations present in the cfDNA and not present in said germ DNA are considered for the subsequent calculation of the instability index.

15. The method according to claim 14, wherein said germ DNA is sequenced with the same reading degree of the sequencing of the cited cfDNA.

16. The method of claim 14, wherein said germ DNA derives from the same liquid biopsy from which the cited cfDNA has been taken.

17. A system for searching and identifying a genetic condition prodromal of the onset of solid tumors in a healthy subject, comprising a set of instructions of computer program aimed at carrying out an evaluation cycle of a genetic stability or instability condition and at least one repetition cycle of said evaluation defined according to claim 1.

**Patentansprüche**

1. Verfahren zum Suchen und Identifizieren einer genetischen Erkrankung, die ein Vorbote für das Auftreten solider Tumore bei einem gesunden Probanden ist, **gekennzeichnet durch** einen Bewertungszyklus zur Bewertung der genetischen Stabilität oder Instabilität und mindestens einen Wiederholungszyklus einer solchen Bewertung, wobei die Wiederholungszyklen regelmäßig am Probanden durchgeführt werden und jeder Zyklus die folgenden Schritte umfasst:

   - Isolieren von DNA aus dem biologischen Material einer Probe, die dem Probanden entnommen wurde, Amplifizieren und Sequenzieren der isolierten DNA;
   - Überprüfen des Vorhandenseins ausgewählter Mutationen in einem vorbestimmten Satz von Genen der betrachteten Probe, wobei der Satz von Genen und die Mutationen mit dem Auftreten solider Tumoren in Verbindung stehen;
   - wobei der vorbestimmte Satz von Genen und die ausgewählten Mutationen unter Berücksichtigung der Anamnese des Probanden definiert werden, die durch eine historische Familienbefragung gewonnen wurde;
   - wobei der vorbestimmte Satz von Genen entweder eine Untergruppe eines Genpanels oder Hotspots umfasst, die aus bekannten Positionen in Genen bestehen, die bei Krebspatienten häufig mutieren und mit einem oder mehreren soliden Tumoren in Verbindung stehen, oder den gesamten Satz von Genen, die mit soliden Tumoren in Verbindung stehen;
   - Überprüfen der Häufigkeit der für jedes Gen und für jeden Bewertungszyklus festgestellten Mutationen, wobei die Mutationen aus den oben genannten ausgewählten Mutationen ausgewählt werden;
   - Aufzeichnen der für jedes Gen oder jede Gruppe von Genen festgestellten Mutationen und ihrer Häufigkeit;
   - Definieren oder Aktualisieren eines genetischen Instabilitätsindex des Subjekts, entweder insgesamt (IT) oder für ein einzelnes Gen (IG), für jeden Wiederholungszyklus, basierend auf der Häufigkeit der nachgewiesenen Mutationen, wobei der genetische Instabilitätsindex (IT, IG) auch auf der Grundlage der Zunahme der Mutationshäufigkeit im Vergleich zu einem oder mehreren vorherigen Bewertungszyklen definiert wird, wobei der Wert des Gesamtinstabilitätsindex (It) eine Funktion des Durchschnitts der Mutationshäufigkeitsvariationen aller Gene ist und der Wert des Instabilitätsindex für jedes einzelne Gen eine Funktion des Durchschnitts der Mutationshäufigkeitsvariationen jedes einzelnen Hotspots des betrachteten Gens ist;
   - in jedem Wiederholungszyklus die Eintritt des Probanden in einen genetischen Zustand, der ein Vorbote für das Auftreten eines oder mehrerer solider Tumore oder Gruppen solider Tumore ist, auf der Grundlage eines Schwellenwerts (ITs, IGs) des genetischen Instabilitätsindex (IT, IG) zu bewerten, der für jedes einzelne Gen oder jede Gruppe von Genen definiert ist und überschritten wird.

2. Verfahren nach Anspruch 1, wobei der Gesamtgenetische Instabilitätsindex (IT) als die Summe der Beziehungen zwischen einem Wert (AFk), der auf den Anstieg der beobachteten Mutationen für jedes Gen reagiert, und der Anzahl der Gene definiert ist, die unter Berücksichtigung der gesamten Gruppe der überwachten Gene bewertet werden.

3. Das Verfahren nach Anspruch 1, wobei der Index der genetischen Instabilität für ein einzelnes Gen (IG) als die Summe der Beziehungen zwischen einem Wert (AFj), der auf den Anstieg der beobachteten Mutationen für jeden Hotspot reagiert, und der Anzahl der bewerteten Hotspots unter Berücksichtigung der gesamten Gruppe der überwachten Hotspots definiert ist.

4. Das Verfahren nach Anspruch 1, wobei die biologische Probe aus einer Flüssigbiopsie besteht und die Phase der Verifizierung des Vorhandenseins von Mutationen in dem vorbestimmten Satz von Genen an einer DNA-Probe durchgeführt wird, die aus der Flüssigbiopsie isoliert und anschließend amplifiziert und sequenziert wird.

5. Das Verfahren nach Anspruch 4, wobei die Flüssigbiopsie peripheres Blut ist.

6. Das Verfahren nach Anspruch 4, wobei die Flüssigbiopsie Urin oder Rückenmarksflüssigkeit ist.

7. Verfahren nach einem der Ansprüche 4 bis 6, wobei ein Anteil an cfDNA in der zu analysierenden DNA-Probe gesucht wird und wobei das Vorhandensein von Mutationen in dem genannten cfDNA-Anteil überprüft wird.

8. Verfahren nach einem der Ansprüche 4 bis 6, wobei die aus der Flüssigbiopsie isolierte ctDNA ebenfalls in der zu analysierenden DNA-Probe gesucht wird.

9. Verfahren nach Anspruch 8, wobei nach der Identifizierung von zirkulierender ctDNA Adressierungsinformationen an ein Früherkennungssystem ("Early Detection") gesendet werden.

10. Verfahren nach Anspruch 8, wobei nach der Identifizierung von ctDNA in der zu analysierenden DNA-Probe das Vorhandensein von CTC ("Circulating Tumor Cells") in der Flüssigbiopsie gesucht wird.

11. Das Verfahren nach Anspruch 1, wobei der vorbestimmte Satz von Genen und ausgewählten Mutationen unter Berücksichtigung ihrer Verbindung zu bestimmten Tumortypen definiert sind.

12. Ein Verfahren nach Anspruch 1, wobei die Wiederholungsperiode nach jedem Wiederholungszyklus entsprechend einem Wert des Instabilitätsindex (IT, Ig), der im aktuellen Zyklus berechnet wurde, und einem Wert desselben, der in einem oder mehreren vorherigen Zyklen berechnet wurde, neu berechnet wird.

13. Verfahren nach Anspruch 1, wobei in jedem Wiederholungszyklus eine höhere Analysesensitivität in Bezug auf das überwachte Gen oder Genpanel eingestellt wird, nachdem der berechnete Wert für den Instabilitätsindex (IT, Ig) gegenüber dem Wert desselben Index (IT, Ig), der in einem oder mehreren vorherigen Zyklen berechnet wurde, angestiegen ist.

14. Verfahren nach Anspruch 1, wobei in jedem der Bewertungs- und Wiederholungszyklen auch die Keimzell-DNA isoliert und sequenziert wird und nur die Mutationen, die in der cfDNA vorhanden sind und nicht in der Keimzell-DNA vorhanden sind, für die anschließende Berechnung des Instabilitätsindex berücksichtigt werden.

15. Verfahren nach Anspruch 14, wobei die Keim-DNA mit dem gleichen Lesegrad wie die Sequenzierung der genannten cfDNA sequenziert wird.

16. Verfahren nach Anspruch 14, wobei die Keim-DNA aus derselben Flüssigbiopsie stammt, aus der die genannte cfDNA entnommen wurde.

17. Ein System zum Suchen und Identifizieren einer genetischen Erkrankung, die ein Vorbote für das Auftreten solider Tumore bei einem gesunden Probanden ist, bestehend aus einer Reihe von Anweisungen eines Computerprogramms, das darauf abzielt, einen Bewertungszyklus einer genetischen Stabilität oder Instabilität und mindestens einen Wiederholungszyklus dieser Bewertung gemäß Anspruch 1 durchzuführen.

**Revendications**

1. Procédé de recherche et d'identification d'une condition génétique prodromique de l'apparition de tumeurs solides chez un sujet sain, **caractérisé en ce qu'**il comprend un cycle d'évaluation de la stabilité ou de l'instabilité génétique et au moins un cycle de répétition de cette évaluation, lesdits cycles de répétition étant effectués périodiquement sur le sujet, chaque cycle comprenant les étapes suivantes :

   - isoler l'ADN du matériel biologique d'un échantillon prélevé sur le sujet, amplifier et séquencer l'ADN isolé ;
   - vérifier la présence de mutations sélectionnées dans un ensemble prédéterminé de gènes de l'échantillon considéré, ledit ensemble de gènes et lesdites mutations étant associés à l'apparition de tumeurs solides ;
   - l'ensemble prédéterminé de gènes et les mutations sélectionnées étant définis en fonction des antécédents médicaux du sujet obtenus par une enquête historique familiale ;
   - l'ensemble prédéterminé de gènes comprenant soit un sous-ensemble d'un panel de gènes ou de points chauds, constitué de positions connues dans des gènes qui mutent fréquemment chez les patients atteints de cancer et qui sont liés à une ou plusieurs tumeurs solides, soit l'ensemble complet des gènes liés aux tumeurs solides ;
   - vérifier la fréquence des mutations détectées pour chaque gène et pour chaque cycle d'évaluation, les mutations étant choisies parmi les mutations sélectionnées susmentionnées ;
   - enregistrer les mutations détectées pour chaque gène ou groupe de gènes et leur fréquence ;
   - définir ou mettre à jour un indice d'instabilité génétique du sujet, soit global (IT), soit pour un gène unique (IG), pour chaque cycle de répétition, sur la base de la fréquence des mutations détectées, ledit indice d'instabilité génétique (IT, IG) étant également défini sur la base de l'augmentation de la fréquence des mutations par rapport à un ou plusieurs cycles d'évaluation précédents, la valeur dudit indice d'instabilité global (It) étant fonction de la moyenne des variations de fréquence des mutations de tous les gènes, et la valeur dudit indice d'instabilité pour chaque gène individuel étant fonction de la moyenne des variations de fréquence des mutations de chaque point chaud individuel du gène considéré ;
   - évaluer, à chaque cycle de répétition, l'entrée du sujet dans une condition génétique prodromique de l'apparition d'une ou plusieurs tumeurs solides ou groupes de tumeurs solides sur la base d'une valeur seuil (ITs, IGs) dudit indice d'instabilité génétique (IT, IG), défini pour chaque gène ou groupe de gènes, qui est dépassée.

2. Procédé selon la revendication 1, dans lequel ledit indice global d'instabilité génétique (IT) est défini comme la somme des relations entre une valeur (AFk) sensible à l'augmentation des mutations observées pour chaque gène et le nombre de gènes évalués en tenant compte de l'ensemble du groupe de gènes surveillés.

3. Procédé selon la revendication 1, dans lequel ledit indice d'instabilité génétique pour un gène unique (IG) est défini comme la somme des relations entre une valeur (AFj) sensible à l'augmentation des mutations observées pour chaque point chaud et le nombre de points chauds évalués, en tenant compte de l'ensemble des points chauds surveillés.

4. Procédé selon la revendication 1, dans lequel l'échantillon biologique consiste en une biopsie liquide, et la phase de vérification de la présence de mutations dans l'ensemble prédéterminé de gènes est réalisée sur un échantillon d'ADN isolé à partir de ladite biopsie liquide, puis amplifié et séquencé.

5. Procédé selon la revendication 4, dans lequel la biopsie liquide est du sang périphérique.

6. Procédé selon la revendication 4, dans lequel la biopsie liquide est de l'urine ou du liquide céphalo-rachidien.

7. Procédé selon l'une des revendications 4 à 6, dans lequel une fraction de ADN libre est recherchée dans l'échantillon d'ADN analysé et dans lequel la présence de mutations est vérifiée dans ladite fraction d'ADN libre.

8. Procédé selon l'une des revendications 4 à 6, dans lequel l'ADNct isolé à partir de la biopsie liquide est également recherché dans l'échantillon d'ADN analysé.

9. Procédé selon la revendication 8, dans lequel, après l'identification de l'ADNct circulant, des informations d'adressage sont envoyées à un système de détection précoce (« Détection précoce »).

**10.** Procédé selon la revendication 8, dans lequel, après l'identification de l'ADNct dans ledit échantillon d'ADN analysé, la présence de CTC (« cellules tumorales circulantes ») est recherchée dans ladite biopsie liquide.

**11.** Procédé selon la revendication 1, dans lequel l'ensemble prédéterminé de gènes et les mutations sélectionnées sont définis en fonction de leur lien avec des types particuliers de tumeurs.

**12.** Procédé selon la revendication 1, dans lequel la période de répétition est recalculée après chaque cycle de répétition en fonction d'une valeur dudit indice d'instabilité (IT, Ig) calculée dans le cycle actuel et d'une valeur de celui-ci calculée dans un ou plusieurs cycles précédents.

**13.** Procédé selon la revendication 1, dans lequel une sensibilité d'analyse plus élevée liée au gène ou au panel de gènes surveillés est définie dans chaque cycle de répétition, à la suite d'une augmentation de la valeur calculée pour ledit indice d'instabilité (IT, Ig) par rapport à la valeur du même indice (IT, Ig) calculée dans un ou plusieurs cycles précédents.

**14.** Procédé selon la revendication 1, dans lequel, dans chacun desdits cycles d'évaluation et de répétition, l'ADN germinal est également isolé et séquencé, et seules les mutations présentes dans l'ADN libre et non présentes dans ledit ADN germinal sont prises en compte pour le calcul ultérieur de l'indice d'instabilité.

**15.** Procédé selon la revendication 14, dans lequel ledit ADN germinal est séquencé avec le même degré de lecture que le séquençage dudit ADN libre.

**16.** Procédé selon la revendication 14, dans lequel ledit ADN germinal provient de la même biopsie liquide que celle à partir de laquelle ledit ADN libre a été prélevé.

**17.** Système permettant de rechercher et d'identifier une condition génétique prodromique à l'apparition de tumeurs solides chez un sujet sain, comprenant un ensemble d'instructions de programme informatique visant à effectuer un cycle d'évaluation d'une condition de stabilité ou d'instabilité génétique et au moins un cycle de répétition de ladite évaluation défini selon la revendication 1.

| ABL1 | AKT1 | ALK | APC | ATM | BRAF | CDH1 | CDKN2A | CSF1R | CTNNB1 |
| EGFR | ERBB2 | ERBB4 | EZH2 | FBXW7 | FGFR1 | FGFR2 | FGFR3 | FLT3 | GNA11 |
| GNAS | GNAQ | HNF1A | HRAS | IDH1 | JAK2 | JAK3 | IDH2 | KDR | KIT |
| KRAS | MET | MLH1 | MPL | NOTCH1 | NPM1 | NRAS | PDGFRA | PIK3CA | PTEN |
| PTPN11 | RB1 | RET | SMAD4 | SMARCB1 | SMO | SRC | STK11 | TP53 | VHL |

Fig. 2

**EP 3 615 682 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160102358 A

**Non-patent literature cited in the description**

- **BETTEGOWDA, C. et al.** Detection of circulating tumor DNA in early- and late-stage human malignancies.. *Science translational medicine*, 2014, vol. 6, 224-224
- **BERT VOGELSTEIN** ; **NICKOLAS PAPADOPOULOS** ; **VICTOR E. VELCULESCU** ; **SHIBIN ZHOU** ; **LUIS A. DIAZ, JR.** ; **KENNETH W. KINZLER\***. *Cancer Genome Landscapes Science*, 29 March 2013, vol. 339 (6127), 1546-1558
- **ISRAEL GOMY**. Hereditary Cancer Risk Assessment: New Perspectives and Challenges for the Next-Gen Sequencing Era. *Front Oncol.*, 2016, vol. 6, 133
- **PEDRO VIANA BAPTISTA**. Principles in genetic risk assessment. *Ther Clin Risk Manag.*, March 2005, vol. 1 (1), 15-20
- **WANG E.** *Predictive genomics: A cancer hallmark network framework for predicting tumor clinical phenotypes using genome sequencing data*
- **MANDEL P** ; **METAIS P**. Les acides nucleiques du plasma sanguin chez l' homme [The nucleic acids in blood plasma in humans].. *C R Seances Soc Biol Fil.*, 1948, vol. 142 (3-4), 241-243
- **STROUN M** ; **ANKER P** ; **MAURICE P** ; **LYAUTEY J** ; **LEDERREY C** ; **BELJANSKI M**. . Neoplastic characteristics of the DNA found in the plasma of cancer patients.. *Oncology.*, 1989, vol. 46 (5), 318-322
- **SAUSEN M** ; **PHALLEN J** ; **ADLEFF V** ; **JONES S** ; **LEARY RJ** ; **BARRETT MT** ; **ANAGNOSTOU V** ; **PARPART-LI S** ; **MURPHY D** ; **KAY LI Q**. Clinical implications of genomic alterations in the tumour and circulation of pancreatic cancer patients.. *Nat Commun.*, 07 July 2015, vol. 6, 7686
- **HAO TB** ; **SHI W** ; **SHEN XJ et al.** Circulating cell-free DNA in serum as a biomarker for diagnosis and prognostic prediction of colorectal cancer.. *Br J Cancer.*, 2014, vol. 111 (8), 1482-1489
- **ZONTA E** ; **NIZARD P** ; **TALY V**. Assessment of DNA integrity, applications for cancer research. *Adv Clin Chem.*, 2015, vol. 70, 197-246
- **CHEN, C.W.** ; **THOMAS, C.A. JR.** Recovery of DNA segments from agarose gels.. *Anal. Biochem.*, 1980, vol. 101, 339-41

- **MARKO, M.A. et al.** A procedure for the large-scale isolation of highly purified plasmid DNA using alkaline extraction and binding to glass powder.. *Anal. Biochem.*, 1982, vol. 121, 382-7
- **BOOM, R. et al.** Rapid and simple method for purification of nucleic acids.. *J. Clin. Microbiol.*, 1990, vol. 28, 495-503
- **MELZAK, K.A. et al.** Driving forces for DNA adsorption to silica in perchlorate solutions.. *J. Colloid Interface Sci. (USA)*, 1996, vol. 181, 635-44
- **JONES S** ; **ANAGNOSTOU V** ; **LYTLE K** ; **PARPART-LI S** ; **NESSELBUSH M** ; **RILEY DR** ; **SHUKLA M** ; **CHESNICK B** ; **KADAN M** ; **PAPP E et al.** Personalized genomic analyses for cancer mutation discovery and interpretation.. *Science translational medicine*, 2015, vol. 7 (283), 283-253
- **NG CK** ; **PISCUOGLIO S** ; **GEYER FC** ; **BURKE KA** ; **PAREJA F** ; **EBERLE C** ; **LIM R, NATRAJAN R** ; **RIAZ N** ; **MARIANI O et al.** The Landscape of Somatic Genetic Alterations in Metaplastic Breast Carcinomas.. *Clinical cancer research : an official journal of the American Association for Cancer Research*, 2017
- **GAGAN J** ; **VAN ALLEN EM**. Next-generation sequencing to guide cancer therapy.. *Genome medicine*, 2015, vol. 7 (1), 80
- **AUTON A** ; **BROOKS LD** ; **DURBIN RM** ; **GARRISON EP** ; **KANG HM** ; **KORBEL JO** ; **MARCHINI JL** ; **MCCARTHY S** ; **MCVEAN GA et al.** A global reference for human genetic variation.. *Nature*, 2015, vol. 526 (7571), 68-74
- **ASHWORTH A** ; **LORD CJ** ; **REIS-FILHO JS**. Genetic interactions in cancer progression and treatment.. *Cell*, 2011, vol. 145 (1), 30-38
- **RICHARDS S** ; **AZIZ N** ; **BALE S** ; **BICK D** ; **DAS S** ; **GASTIER-FOSTER J** ; **GRODY WW** ; **HEGDE M** ; **LYON E** ; **SPECTOR E et al.** Standards and guidelines for the interpretation of sequence variants: a joint consensus recommendation of the American College of Medical Genetics and Genomics and the Association for Molecular Pathology.. *Genetics in medicine : official journal of the American College of Medical Genetics*, 2015, vol. 17 (5), 405-424

- **DE MATTOS-ARRUDA L** ; **CALDAS C**. Cell-free circulating tumour DNA as a liquid biopsy in breast cancer.. *Molecular oncology*, 2016, vol. 10 (3), 464-474
- **DIAZ LA, JR.** ; **BARDELLI A**. Liquid biopsies: genotyping circulating tumor DNA.. *Journal of clinical oncology : official journal of the American Society of Clinical Oncology*, 2014, vol. 32 (6), 579-586
- **PANTEL K** ; **DIAZ LA, JR.** ; **POLYAK K**. Tracking tumor resistance using 'liquid biopsies'.. *Nature medicine*, 2013, vol. 19 (6), 676-677
- **GARRAWAY LA** ; **VERWEIJ J** ; **BALLMAN KV**. Precision oncology: an overview.. *Journal of clinical oncology : official journal of the American Society of Clinical Oncology*, 2013, vol. 31 (15), 1803-1805
- **YOHE SL** ; **CARTER AB** ; **PFEIFER JD** ; **CRAWFORD JM** ; **CUSHMAN-VOKOUN A** ; **CAUGHRON S** ; **LEONARD DG**. Standards for Clinical Grade Genomic Databases. *Archives of pathology & laboratory medicine*, 2015, vol. 139 (11), 1400-1412
- **AABOUD M** ; **AAD G** ; **ABBOTT B** ; **ABDALLAH J** ; **ABDINOV O** ; **ABELOOS B** ; **ABEN R** ; **ABOUZEID OS** ; **ABRAHAM NL** ; **ABRAMOWICZ H et al.** Search for triboson [Formula: see text] production in pp collisions at [Formula: see text] [Formula: see text] with the ATLAS detector.. *The European physical journal C, Particles and fields*, 2017, vol. 77 (3), 141
- **DACHEVA D** ; **DODOVA R** ; **POPOV I** ; **GORANOVA T** ; **MITKOVA A** ; **MITEV V** ; **KANEVA R**. Validation of an NGS Approach for Diagnostic BRCA1/BRCA2 Mutation Testing.. *Molecular diagnosis & therapy*, 2015, vol. 19 (2), 119-130
- **BETTEGOWDA C** ; **SAUSEN M** ; **LEARY RJ** ; **KINDE I** ; **WANG Y** ; **AGRAWAL N** ; **BARTLETT BR** ; **WANG H** ; **LUBER B** ; **ALANI RM et al.** Detection of circulating tumor DNA in early- and late-stage human malignancies.. *Science translational medicine*, 2014, vol. 6 (224), 224-224